# FASCICULE DE BREVET EUROPEEN

(11) **EP 1 343 453 B1**
(45) Date de publication et mention de la délivrance du brevet: **14.05.2008**
(21) Numéro de dépôt: 01989636.4
(22) Date de dépôt: 18.12.2001
(51) Int. Cl.: A61K 6/093, C08K 5/55, C08G 77/08

(54) **COMPOSITION DENTAIRE A BASE D'UNE SILICONE FONCTIONNALISEE RETICULABLE ET/OU POLYMERISABLE PAR VOIE THERMIQUE**
DENTALZUSAMMENSETZUNG AUF BASIS EINES FUNKTIONALISIERTEN SILIKONS, DAS VERNETZBAR UND/ODER DURCH EIN WÄRMEVERFAHREN POLYMERISIERBAR IST
DENTAL COMPOSITION BASED ON A FUNCTIONALISED SILICONE CROSSLINKABLE AND/OR POLYMERISABLE BY HEAT-PROCESS

(30) Priorité: 22.12.2000 FR 0016922
(43) Date de publication de la demande: 17.09.2003
(73) Titulaire: Bluestar Silicones France, 69486 Lyon (FR)
(72) Inventeur: FRANCES, Jean-Marc, F-69330 MEYZIEU (FR)
(86) Numéro de dépôt international: PCT/FR2001/004044
(87) Numéro de publication internationale: WO 2002/051357

(56) Documents cités:
- EP-A- 0 562 897
- WO-A-00/19966
- WO-A-00/19967
- FR-A- 2 727 416
- CHEMICAL ABSTRACTS, vol. 133, no. 2, 10 juillet 2000 (2000-07-10) Columbus, Ohio, US; abstract no. 17513, PARKS, DANIEL J. ET AL: "Studies on the Mechanism of B(C6F5)3-Catalyzed Hydrosilation of Carbonyl Functions" XP002163707 & J. ORG. CHEM. (2000), 65(10), 3090-3098,

## Description

Le domaine de l'invention est celui des compositions dentaires. Plus précisément, les compositions dentaires mises au point dans le cadre de la présente invention sont à base de silicone fonctionnalisée réticulable et/ou polymérisable, et sont utilisables pour la réalisation de prothèses dentaires, de dents artificielles et de matériaux de restauration dentaire.

A ce jour, pour réaliser des compositions dentaires pour la préparation de prothèses dentaires ou de matériaux de restauration dentaire, on peut utiliser des résines à base d'acrylates photopolymérisables. Ces produits *'prêt-à-formuler'* présentent toutefois à l'utilisation des problèmes d'irritation et des problèmes potentiels de toxicité.

En outre, ces produits présentent l'inconvénient majeur d'engendrer un retrait volumique important lors de leur polymérisation: ce qui rend leur utilisation complexe et difficile pour la réalisation de prothèses dentaires ou de matériaux de restauration dentaire. On observe notamment des problèmes d'accrochage dus au retrait volumique ou au manque d'adhérence des polymères utilisés.

La présente invention a pour objet de fournir de nouvelles compositions dentaires à base de silicone ne présentant pas les inconvénients de l'art antérieur. Ces nouvelles compositions dentaires, polymérisables et/ou réticulables en environnement oral, ont des qualités nettement améliorées, notamment en ce qui concerne la réduction très nette du phénomène de retrait des matériaux et prothèses obtenus à partir desdites compositions.

Ces nouvelles compositions dentaires sont particulièrement intéressantes en terme de réactivité; elles sont réticulables et/ou polymérisables par voie thermique, ce qui permet d'utiliser des méthodes de réticulation/polymérisation classiques et de plus à des températures inférieures à 150°C. Cette réactivité est notamment due à la présence d'un nouveau type d'amorceur à base de dérivé de bore; en effet, celui-ci est actif à faible concentration et ne nécessite avantageusement que de faibles quantités d'énergie pour effectuer la réticulation/polymérisation.

De ce fait, les compositions dentaires revendiqués se révèlent donc particulièrement avantageuses en termes d'efficacité à l'usage et en termes de rentabilité et de coût pour les procédés industriels.

Le WO 00/19967 A décrit des compositions dentaires comprennent une silicone réticulable et/ou polymérisable par voie cationique, un amorceur de type borate d'onium, au moins un photosensibilisateur et une charge dentaire.

Le WO 01/74938 A, document relève de l'art. 54(3) CBE, concerne l'utilisation titre de catalyseur thermoactivable d'au moins un dérivé de bore de formule (A)ₓB(R')_{y} pour la déshydrogénocondensation entre, d'une part, au moins un monomère, oligomère et/ou polymère organosiloxane ayant, par molécule.,au moins un motif réactif SiH et, d'autre part, au moins un monomère, oligomère et/ou polymère organosiloxane présentant, par molécule, au moins un motif réactif SiOH.

La composition dentaire polymérisable et/ou réticulable thermiquement selon l'invention comprend :
**(1)** 1 à 99 % en poids, et de préférence de 5 à 50%, d'au moins un oligomère ou polymère silicone réticulable et/ou polymérisable, liquide à température ambiante ou thermofusible à température inférieure à 100°C, et comprenant :
   - au moins un motif de formule (FS) : 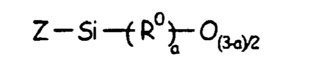 dans laquelle :
      - a = 0, 1 ou 2,
      - R⁰ , identique ou différent, représente un radical alkyle, cycloalkyle, aryle, vinyle, hydrogéno, alcoxy, de préférence un alkyle inférieur en C₁-C₆,
      - Z, identique ou différent, est un substituant organique comportant au moins une fonction réactive époxy, et/ou alcénylether et/ou oxétane et/ou dioxolane et/ou carbonate, et de préférence Z étant un substituant organique comportant au moins une fonction réactive époxy, et/ou dioxolane,
   - et au moins deux atomes de silicium,
**(2)** de 5 à 90 % en poids, et de préférence 10 à 80 %, d'au moins une charge dentaire,
**(3)** de 0,001 à 5% en poids d'au moins un amorceur de polymérisation et/ou de réticulation activable thermiquement comprenant un dérivé du bore de formule (I) et/ou sa ou ses formes solvatées:

   (A)ₓB(R')_{y} (I)

   dans laquelle
   · les symboles R' sont identiques ou différents et représentent :
      - un radical alkoxy en C₁-C₁₂, de préférence en C₁-C₈, linéaire ou ramifié, éventuellement substitué par au moins un élément électroattracteur, notamment un atome d'halogène (le fluor tout particulièrement), ou un groupement électroattracteur comme par exemple les groupements CF₃, NO₂, CN,
      - un radical phényle substitué par au moins (i) un élément électroattracteur, notamment un atome d'halogène (fluor tout particulièrement), (ii) un groupement électroattracteur, notamment un groupement CF₃, NO₂, CN, ou (iii) un radical hydrocarboné en C₁-C₁₂, de préférence en C₁-C₈, linéaire, ramifié, mono- ou poly- cyclique, saturé, insaturé ou aromatique et de préférence substitué par au moins un élément atome d'halogène ou un radical alkyle en C₁-C₁₂, linéaire ou ramifié, mono- poly- ou per-halogéné, et
      - un radical aryle contenant au moins deux noyaux aromatiques tel que biphényle, naphtyle, éventuellement substitué par au moins un élément électroattracteur, notamment atome d'halogène (fluor tout particulièrement), ou un groupement électroattracteur notamment un groupement CF₃, NO₂, CN,
      - deux groupements R' peuvent être liés entre eux de manière à constituer avec l'atome de bore auquel ils sont liés un cycle à 5 ou 10 atomes avec ledit cycle pouvant être saturé, insaturé ponté et/ou aromatique et comprendre un ou plusieurs hétéroatomes choisis parmi les atomes d'hydrogène, d'azote et de bore avec l'atome bore présent dans ledit cycle pouvant lui-même être substitué par un radical tel que défini pour A ou R' en formule générale I;
   · les symboles A représentent indépendamment l'un de l'autre :
      - un atome d'halogène, ou
      - un radical hydroxyle;
   · x représente 0 ou l'entier 1 ou 2 et y l'entier 1, 2 ou 3 avec la somme de x+y étant égale à 3.

En général, l'activation thermique est réalisée à des températures inférieures à 150°C, de préférence inférieure à 100°C et voire notamment à température ambiante de mise en bouche.

Les amorceurs dérivés du bore utilisés au sein des compositions dentaires conformes à l'invention sont de manière générale des composés très hygroscopiques. En conséquence, ces composés peuvent se présenter sous l'aspect de mélange entre le composé tel que défini en formule générale (1) et sa ou ses différente(s) forme(s) hydratée(s). De même, lors de la formulation de cet amorceur avec un solvant, on observe la formation de dérivés solvatés. Ce phénomène peut s'observer avec des solvants aprotiques tels les éthers, esters et silicones ou des solvants protiques comme les alcools, acides carboxyliques, silanols, amines, thiols, l'eau ou leurs mélanges.

En conséquence, la présente invention s'étend également à ces formes solvatées.

Ces amorceurs peuvent en outre être associés à un amorceur conventionnel tel qu'un photoamorceur cationique. Ceci est particulièrement avantageux en terme de rentabilité, dans la mesure où il s'avère ainsi possible de diminuer significativement la quantité efficace en amorceur conventionnel de ce type. La réticulation et/ou polymérisation est par ailleurs réalisée totalement.

Plus préférentiellement, les symboles R' de la formule générale (I) sont choisis de manière à conférer à l'atome de bore auquel ils sont liés un encombrement stérique suffisant pour lui assurer une protection efficace notamment pour prévenir son oxydation et/ou hydratation. En l'espèce, les amorceurs de formule générale (I) dans laquelle au moins un des symboles R' et de préférence au moins deux d'entre eux représentent un radical phényle ou aryle sont particulièrement intéressants.

De même, il est avantageux que les symboles R' soient substitués et notamment par des éléments et/ou groupements électroattracteurs de manière à assurer audit atome de bore une électronégativité qui soit compatible avec ses propriétés électrophiles. C'est ainsi que s'avèrent particulièrement efficaces des amorceurs de formule générale (I) dans laquelle les symboles R' contribuent globalement avec les symboles A à un σₚ au moins égal à celui de 3 radicaux (C₆H₄F).

Sont notamment préférés selon l'invention, les amorceurs répondant à la formule générale (Ia) : 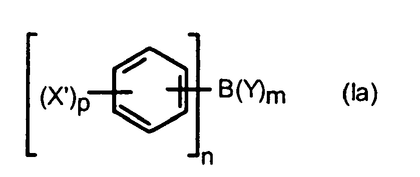 dans laquelle:
- n représente un nombre entier compris entre 1 et 3 et m un nombre entier compris entre 0 et 2 avec la somme de n et m étant égale à 3,
- les symboles Y sont identiques ou différents et représentent
   a) un groupement hydroxyle,
   b) un atome d'halogène,
   c) un radical alkoxy en C₁-C₁₂, de préférence en C₁-C₈, linéaire ou ramifié, de préférence substitué par au moins un élément électroattracteur comme un atome d'halogène et en particulier un atome de fluor,
   d) deux groupements Y peuvent être liés entre eux de manière à constituer avec l'atome de bore auquel ils sont liés un cycle en C₅-C₁₀ avec ledit cycle pouvant être saturé, insaturé, ponté et/ou aromatique et comprendre un ou plusieurs hétéroatomes choisis parmi les atomes d'oxygène, d'azote et de bore avec l'atome bore présent dans ledit cycle pouvant lui-même être substitué par un radical tel que défini pour Y en formule générale (la) et
- les symboles X' sont identiques ou différents et représentent
   - un atome d'halogène de préférence un atome de fluor,
   - un radical hydrocarboné en C₁-C₁₂, de préférence en C₁-C₈, linéaire, ramifié, mono- ou poly- cyclique, saturé, insaturé ou aromatique et de préférence substitué par au moins un élément électroattracteur comme un atome d'halogène et en particulier un atome de fluor ou un radical alkyle en C₁-C₁₂, de préférence en C₁-C₈, linéaire ou ramifié, mono- poly- ou per-halogéné avec en particulier le fluor comme atome d'halogène, et
- les indices p sont identiques ou différents et représentent un entier compris entre 0 et 5, avec de préférence au moins l'un des symboles p supérieur à 3 et plus préférentiellement égal à 5.

Les amorceurs de formule générale (Ia) dans laquelle Y répond aux définitions a), b), et c) sont particulièrement intéressants.

A titre représentatif des amorceurs revendiqués, on peut plus particulièrement citer les composés suivants : 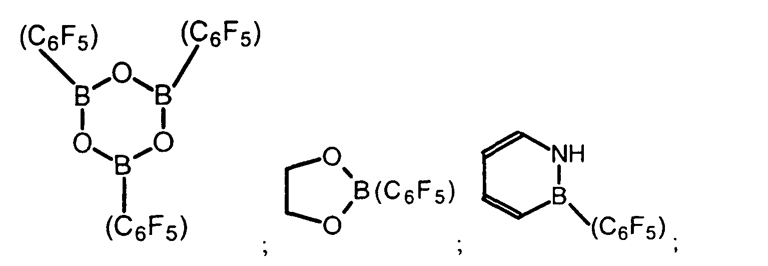 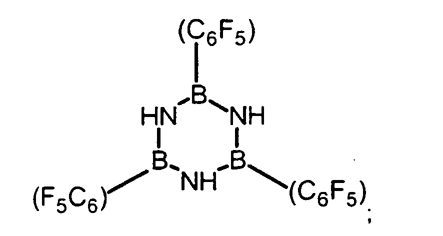 (C₅F₄)(C₆F₅)₂B ; (C₅F₄)₃B ; (C₆F₅)BF₂ : BF(C₆F₅)₂ ; B(C₆F₅)₃ ;
BCl₂(C₆F₅) ; BCl(C₆F₅)₂ ; B(C₆H₅)(C₆F₅)₂; B(C₆H₅)₂(C₆F₅); 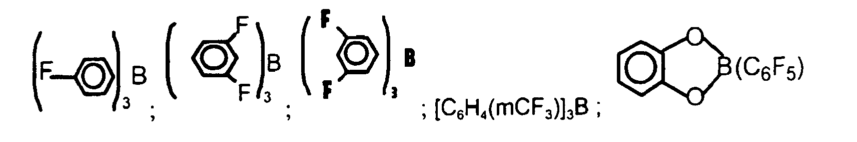 [C₅H₄(pOCF₃)]₃B ; (C₆F₅)B(OH)₂ ; [C₆H₃bis(mCF₃)₂]₃B; (C₇H₁₁)B(C₆F₅)₂; 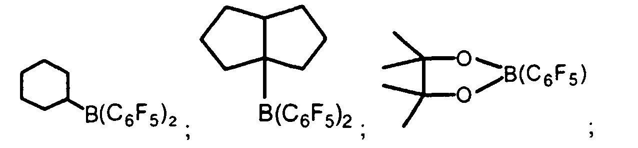 (C₆F₅)₂B(OC₂H₅) ; (C₆F₅)₂ BOH ;

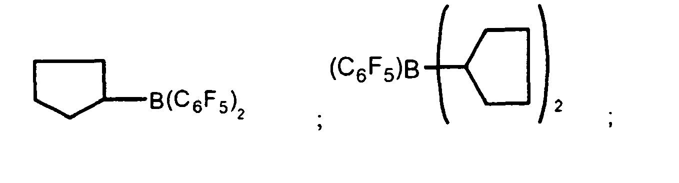

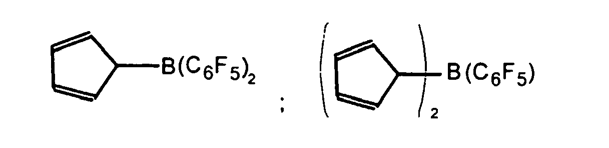

Les amorceurs utilisés au sein des compositions selon l'invention peuvent être mis en oeuvre, tels qu'ils sont obtenus à l'issue de leur procédé de préparation, par exemple sous forme solide ou liquide, ou en solution dans au moins un solvant approprié. Le terme solvant englobe présentement les produits solubilisant les amorceurs solides et les produits diluant les amorceurs liquides ou solides.

De préférence, les amorceurs sont généralement mis en oeuvre en solution dans un solvant. Les proportions pondérales entre le ou les amorceurs, d'une part, et le solvant, d'autre part, sont comprises entre 0.1 et 99 parties pour 100 parties de solvant et, de préférence de 10 à 50 parties.

Cette solution d'amorceur est donc utilisée pour préparer un bain avec le ou les oligomères et/ou polymères silicones à groupements fonctionnels réticulables, telle que la concentration de l'amorceur ou des amorceurs présents soit comprise entre 0.01 et 5% en poids dans ledit bain, et de préférence entre 0.05 et 0.5%.

Les solvants utilisables pour les amorceurs sont très nombreux et variés et sont choisis selon l'amorceur utilisé et les autres constituants de la composition de l'invention. En général, les solvants peuvent être des alcools, des esters, des éthers, des cétones, l'eau à l'état de traces et les carbonates.

Les alcools couramment employés sont le para-tolyl-éthanol, l'isopropyl-benzyl-alcool, l'alcool benzylique, le méthanol, l'éthanol, le propanol, l'isopropanol et le butanol. Les éthers communément usités sont le méthoxy-2-éthanol, l'éthoxy-2-éthanol, le diéthylène-glycol. Les esters usuels sont le dibutylmaléate, diméthyl-éthytmalonate, salycilate de méthyle, dioctyladipate, tartrate de butyle, lactate d'éthyle, lactate de n-butyle, lactate d'isopropyle. D'autres solvants utilisables pour le bain de l'amorceur et entrant dans les autres catégories de solvants citées ci-dessus sont l'acétonitrile, le benzonitrile, l'acétone, le cyclohexanone, et le tétrahydrofurane.

En outre, parmi les solvants utilisables pour mettre en solution le ou les amorceurs. certains types de solvants organiques donneurs de protons ainsi que certains types d'esters d'acides carboxyliques hydroxylés ont pour propriétés également d'améliorer significativement leurs performances de réactivité et de cinétique.

Comme signalé précédemment, en solution l'amorceur au sein des compositions selon l'invention peut évoluer vers une forme solvatée.

Les différentes formes peuvent coexister au sein du solvant sous l'effet d'un équilibre. A titre représentatif de ces phénomènes de solvatation, on peut notamment citer :

| | |
|---|---|
| eau | H₂O + B(Ar)₃ ⇄ H⁺[B(Ar)₃(OH)]⁻ |
| alcool | R*OH + B(Ar)₃ ⇄ H⁺[B(Ar)₃(OR*)]⁻ |
| amine | R*₂NH + B(Ar)₃ ⇄ H⁺[B(Ar)₃(NR*₂)]⁻ |
| amine | R⁺₃N + B(Ar)₃ ⇄ B(Ar)₃(NR*₃) |
| acide | R*COOH+ B(Ar)₃ ⇄ H⁺[B(Ar)₃(OCOR*)]⁻ |

Le type d'amorceur utilisé au sein des compositions selon l'invention s'étend également aux formes solvatées des amorceurs revendiqués.

Dans le cadre de l'invention, les fonctions réactives Z du polymère ou oligomère silicone (1) peuvent être très variées.

Toutefois, selon une disposition intéressante de l'invention, des compositions dentaires particulièrement intéressantes sont obtenues lorsque l'oligomère ou polymère silicone (1) comprend au moins un motif (FS) dans lequel Z représente un substituant organique Z1 comportant au moins une fonction réactive époxy, et/ou dioxolane, et de préférence au moins une fonction réactive époxy.

Selon deux alternatives avantageuses de la présente invention, l'oligomère ou polymère silicone (1) avec au moins une fonction réactive Z1 époxy, et/ou dioxolane, et de préférence au moins une fonction réactive époxy peut :
(i) soit comporter uniquement ce(s) type(s) de fonction(s) réactive(s) Z1,
(ii) ou soit comporter d'autres fonctions réactives Z telles que les fonctions réactives Z2 alcénylether, oxétane et/ou carbonate.

Dans le cas de la première alternative (i), la composition dentaire peut également comprendre d'autres oligomères et/ou polymères silicones comportant d'autres fonctions réactives Z2 telles que les fonctions alcénylether, oxétane et/ou carbonate et éventuellement des fonctions réactives Z1.

A titre d'exemples de fonctions réactives Z, celles-ci peuvent être notamment choisies parmi les radicaux suivants : 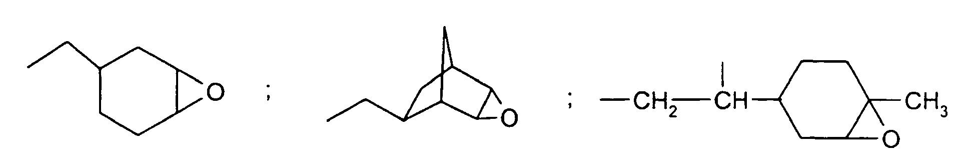 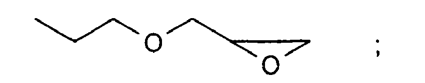 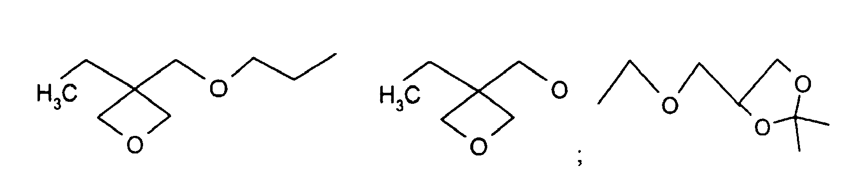 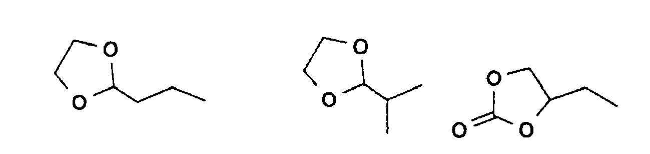 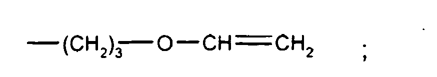 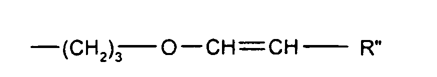
- avec R" représentant un radical alkyle linéaire ou ramifié en C₁-C₆.

A titre d'exemples, le polymère ou oligomère silicone est constitué par au moins une silicone de formule moyenne suivante :
a) 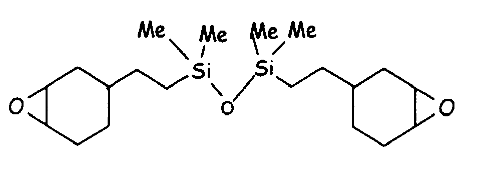
b) 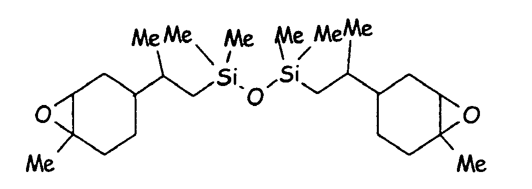
c) 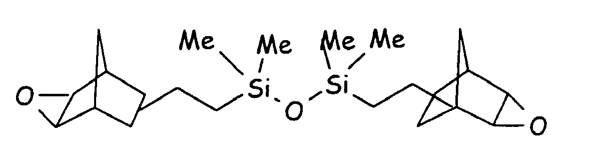
d) 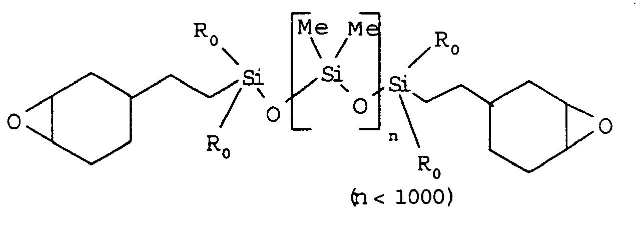
e) 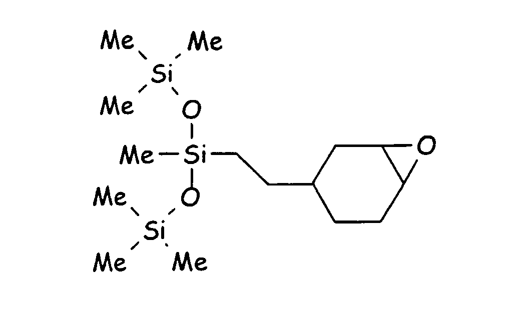
f) 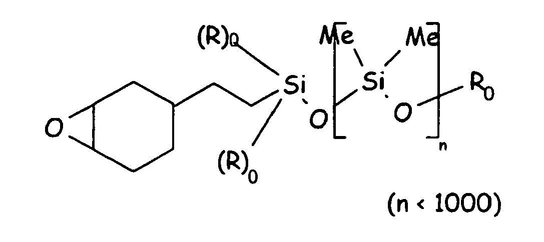
9) 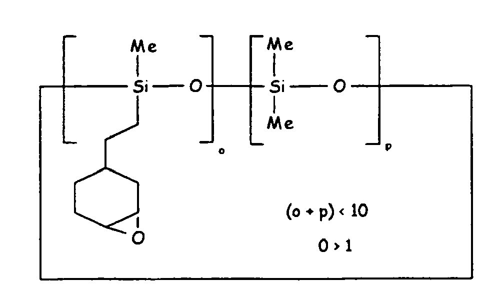
h) 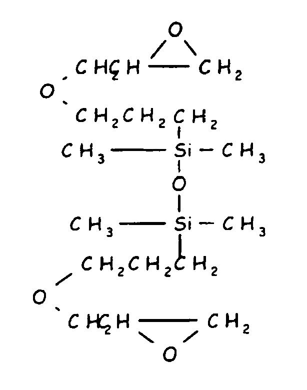
i) 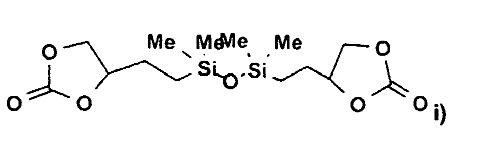
j) 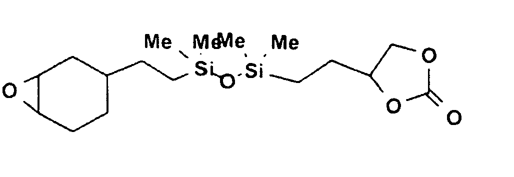
k) 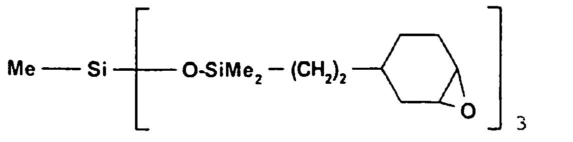
l) 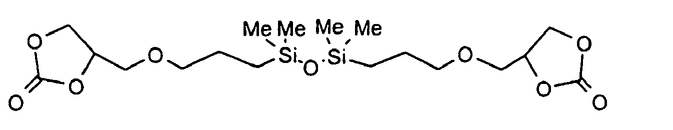
m) 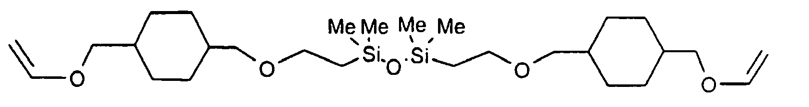
n) 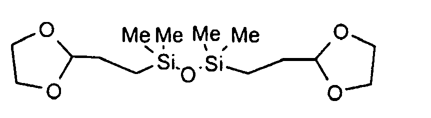
o) 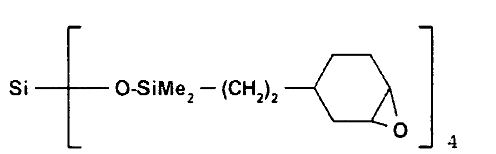
p) 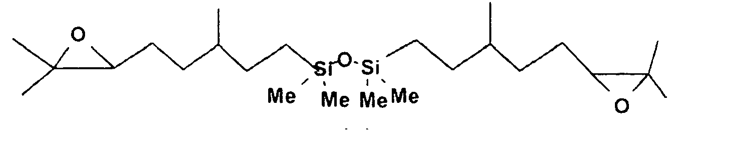
q) 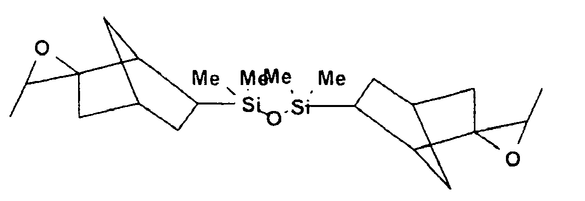
r)

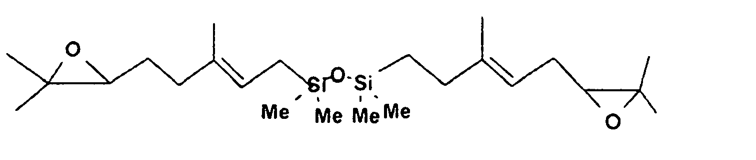

Selon une disposition intéressante de l'invention, outre des oligomères et/ou polymères de nature silicone, la composition dentaire peut comprendre des monomères, oligomères et/ou polymères réticulables et/ou polymérisables de nature organique. Ceux-ci peuvent être notamment choisis parmi les espèces organiques suivantes:
α**_{1.1)}** les époxydes cycloaliphatiques et notamment :
   - les époxydes du type 3,4-époxycyclohexylméthyl-3',4'-époxycyclohexane carboxylate : 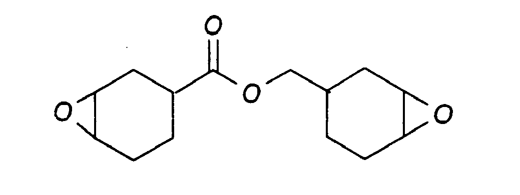
   - le Bis(3,4-époxycyclohexyl)adipate,
   - les époxydes de type glycidiques tels que : le néopentylglycol, diglycidylether,le dibutanediol1-4 difglycidyléther,le glycidyléther dodécyl,,le cyclohexanedimethanoldiglycidylether,le trimethylolpropanetriglycidylether,
      - et les époxydes décrits dans les documents US, 6,084,111 et US 6,075,155;
α**_{1.2)}** les époxydes non cycloaliphatiques, et notamment:
   - les époxydes du type de ceux résultant de la condensation de Bis-phénol A et d'épichlorhydrine et du type :
      - di et triglycidyléthers de Bisphénol A alcoxylé de 1,6 hexane diol, de glycérol, de néopentylglycol et de propane triméthylol,
      - ou diglycidyléthers de Bisphénol A,
   - les époxydes d'alpha-oléfines, époxydes NOVOLAC, huile de soja époxydé, huile de lin époxydé, et polybutadiène époxydé;
α**₂₎** les oxydes de terpènes, et notamment : le dioxyde de limonène ; le myrcène6-7 époxyde ou le dioxyde de myrcène , le dihydromyrcène 6-7 époxyde ; l'oxyde de pinène ; le 5-ethylidenenorbornene 5-8 époxyde ; et le 5-vinyl 2,3 epoxynorbornene ;
α**₃₎** les alcényl-éthers, linéaires ou cycliques, et notamment :
   - les vinyl-éthers, en particulier l'octylvinyléther, le dodécylvinyléther (DDVE), le cyclohexylvinyléther (CVE), le butanediol divinyléther (BDVE), le butanediol monovinyléther (HBVE), le cyclohexane diméthanol divinyléther (CHDVE), le cyclohexane diméthanol monovinyléther (CHMVE), le triéthylèneglycol divinyléther (DVE-3) et les vinyléthers de formule : 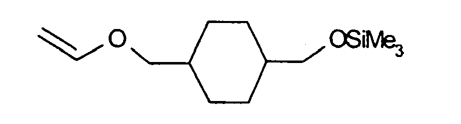 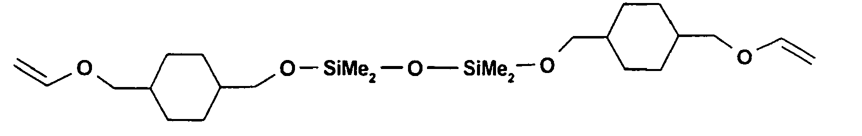
   - les propényl-éthers,
   - et les butényl-éthers ;
α**₄₎** les polyols, et de préférence le composé de formule : 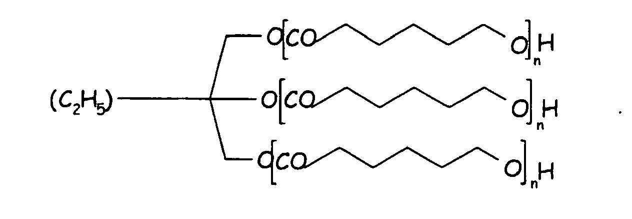
α**₅₎** les oxétanes, et par exemples les oxétanes de formules suivantes :

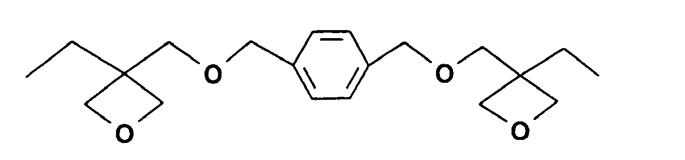

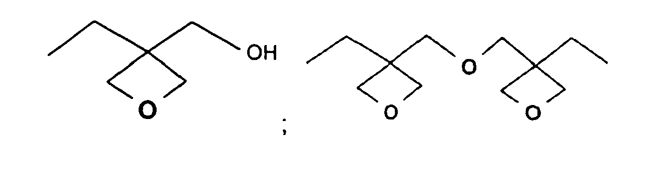

Différents types de charges sont utilisables pour préparer les compositions selon l'invention. Les charges sont choisies en fonction de l'utilisation finale de la composition dentaire : celles-ci affectent d'importantes propriétés telles que l'apparence, ainsi que les propriétés mécaniques et physiques du matériau obtenu après réticulation et/ou polymérisation de la composition dentaire.

Comme charge de renforcement, on peut utiliser des charges de silice de pyrogénation traitée ou non, des charges de silice amorphe, du quartz, des verres ou des charges non vitreuses à base d'oxydes de zirconium, de bore, de baryum, de calcium, de fluor, d'aluminium, de titane, de strontium, de zinc, des borosilicates, des aluminosilicates, du talc, des sphérosil, du trifluorure d'yterbium, des charges à base de polymères sous forme de poudre broyée telles que des polyméthacrylates de méthyle inertes ou fonctionnalisés, des polyépoxydes ou des polycarbonates.

A titre d'exemple, on citera :
- des charges inertes à base de polyméthacrylate de méthyle LUXASELF de la société UGL utilisables dans le domaine dentaire et pigmentées en rose,
- des charges de silice de combustion traitée hexaméthyldisilazane de surface spécifique 200 m²/g,
- des charges de silice de combustion non traitée (« aerosil » AE200 commercialisée par DEGUSSA).

Selon une variante avantageuse de l'invention, les charges et en particulier les charges de silice, sont traitées avant utilisation à 120°C avec une quantité inférieure à 10% p/p de silicone comprenant au moins un motif de formule ci-dessous : 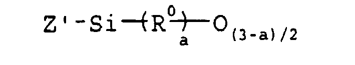
- tel que Z' a la même définition que Z
- a=0,1,2ou3
- avec au moins un atome de silicium.

On peut citer à titre d'exemple, le polymère décrit ci-dessous avec Z = époxyde et Z = trialcoxysilyle :

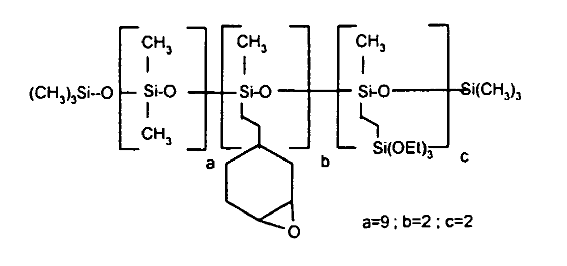

Dans ce cas de traitement de ou des charges siliciées en particulier la silice avec ce type de polymère, le matériau obtenu après réticulation présente une tenue mécanique, un module d'élasticité, et une résistance à la compression nettement améliorés.

Outre les charges de renforcement, des pigments peuvent être utilisés pour teinter la composition dentaire selon l'utilisation envisagée et les groupes ethniques.
Par exemple, on utilise des pigments rouges en présence de microfibres pour les compositions dentaires utilisées pour la préparation de prothèses dentaires afin de simuler les vaisseaux sanguins.
On emploie aussi des pigments à base d'oxydes métalliques (oxydes de fer et/ou titane et/ou aluminium et/ou zirconium, etc.) pour les compositions dentaires utilisées pour la préparation de matériau de restauration, afin d'obtenir un matériau réticulé de couleur ivoire ou blanche.

D'autres additifs peuvent être incorporés au sein des compositions dentaires selon l'invention. Par exemple, des biocides, des stabilisants, des agents de flaveur, des plastifiants et des promoteurs d'adhérence.

Parmi les additifs envisageables, on utilisera avantageusement des co-réactifs réticulables et/ou polymérisables de type organique. Ces co-réactifs sont liquides à température ambiante ou thermofusibles à température inférieure à 100°C, et chaque co-réactif comprend au moins deux fonctions réactives telles que oxétane-alcoxy, oxétane-hydroxy, oxétane-alcoxysilyle, carboxy-oxétane, oxétane-oxétane, alcénylether-hydroxy, alcénylether-alcoxysilyle, époxy-alcoxy, époxy-alcoxysilyles, dioxolane-dioxolane- alcool, etc.

Les compositions dentaires selon l'invention sont utilisables dans le domaine de la prothèse dentaire, le domaine du matériau de restauration dentaire, et le domaine de la dent artificielle. Elles peuvent être formulées sous forme monocomposante ou sous forme bicomposante.

Dans le cadre de l'invention :
- les compositions dentaires pour prothèse dentaire comprennent de 25 à 90 % en poids de silicone et de 10 à 75 % de charge(s) ainsi qu'une quantité efficace de l'amorceur de formule (I),
- les compositions dentaires pour dent artificielle comprennent de 5 à 50 % en poids de silicone et de 50 à 95 % de charge(s) ainsi qu'une quantité efficace de l'amorceur de formule (I),
- les compositions dentaires pour la restauration dentaire comprennent de 15 à 50 % en poids de silicone et de 50 à 95 % de charge(s) ainsi qu'une quantité efficace de l'amorceur de formule (I).

Les préparations pour prothèse dentaire, dent artificielle, et matériau de restauration dentaire sont effectuées selon les techniques usuelles du métier.

La stabilité avant réticulation et/ou polymérisation des compositions dentaires monocomposantes ou bicomposantes peut être assurée par des dérivés à fonctions amines, notamment des amines stériquement encombrées telles que les amines de type HALS. On peut utiliser en particulier les amines enseignées dans le document WO 98/07798.

Il est à noter que les produits obtenus à partir de la composition dentaire selon l'invention sont non poreux. Ainsi, après un éventuel polissage à l'aide d'une brosse feutre par exemple, la surface des prothèses dentaires obtenues est lisse et brillante et donc ne nécessite pas d'utilisation de vernis.

L'application dans le domaine de la prothèse dentaire est essentiellement celle de la prothèse adjointe, que l'on peut diviser en deux types :
- prothèse totale (maxillaires supérieur et inférieur) en cas de patient complètement édenté,
- et prothèse partielle due à l'absence de plusieurs dents se traduisant par soit une prothèse provisoire, soit un appareil squeletté.

Cette application concerne aussi la réalisation de bords et la réparation de prothèse (rebasage par coulée ou injection).

Dans le cas d'application de la composition dentaire à une dent, soit la dent peut être pré-traitée avec un primaire d'accrochage ou soit la composition dentaire peut être préparée en mélange avec un primaire d'accrochage avant son utilisation. Toutefois, il n'est pas indispensable d'utiliser un primaire d'accrochage pour utiliser la composition dentaire selon l'invention.

La composition dentaire étant non toxique et polymérisable en couches épaisses, il n'est pas indispensable de polymériser le matériau en couches successives. En général, une seule injection de la composition dentaire est suffisante.

### Exemples et Tests.

Les Exemples et Tests suivants sont donnés à titre illustratif. Ils permettent notamment de mieux comprendre l'invention et de faire ressortir certains de ses avantages et d'illustrer quelques-unes de ses variantes de réalisation. Les exemples 1 à 8 et 10 concernent des compositions pré-dentaires [(sans charge(s)] utilisables notamment pour la prothèse dentaire. Les exemples 9 et 11 concernent des compositions dentaires [avec charge(s)].

Les produits utilisés dans les compositions des exemples sont les suivants : 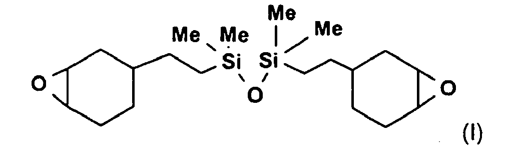 et

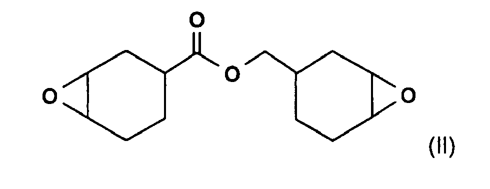

La durée de vie des compositions *"prét-à-réticuler"* est réglée par l'ajout d'une amine organique stériquement encombrée commercialisée sous le nom Tinuvin 765 par la société Ciba. La durée de vie correspond au temps pendant lequel la composition est manipulable.

### Exemples 1 à 6.

Dans chaque exemple, l'oligomère de formule (1) est introduit dans un moule pour prothèses dentaires à raison de 100 g sur une épaisseur de 10 mm. On ajoute ensuite à température ambiante 2,8 g d'une solution isopropanolique de borane B(C₆F₅)₃ à 3% en poids; cette solution contient en outre une amine encombrée stériquement (produit Tinuvin 765®).

Dans chaque exemple, la concentration en amine de la solution de borane est différente. Les rapports molaires R de l'amine exprimée en fonction azote par rapport à celui du borane exprimé en fonction bore sont donnés dans la table 1 ci-dessous.

Pour chaque composition réticulée, on mesure le temps de gel à 20°C et on mesure la dureté Shore D (selon norme DIN 43505) obtenue après déclenchement de la réaction de polymérisation en laissant revenir à température ambiante de 22°C ± 2 après 24 h et après démoulage. Les matériaux obtenus ne présentent pas de porosités.

**Table 1.**

| Exemple | %Borane | %Tinuvin | R | Temps de gel (min) | Dureté shore D (24h) |
|---|---|---|---|---|---|
| 1 | 3 | 0.66 | 0.49 | 0.5 | 85 |
| 2 | 3 | 1.26 | 0.93 | 1 | 85 |
| 3 | 3 | 1.325 | 1 | 3 | 85 |
| 4 | 3 | 1.4 | 1.04 | 3.5 | 85 |
| 5 | 3 | 1.5 | 1.11 | 10 | 85 |
| 6 | 3 | 1.66 | 1.23 | 600 | 85 |

### Exemple 7.

Cet exemple concerne une composition à base de 100 g d'oligomère (I) mélangée avec 2,8 g de solution d'amorceur borane contenant 1,66% en poids de Tinuvin 765 et 3% en poids de borane. La composition est réticulée dans un four à 60°C pendant 10 minutes.

La dureté Shore D mesurée après refroidissement est de 85. Le matériau obtenu est totalement transparent et incolore. Le retrait volumique du matériau sans charges mesuré par pycnométrie est compris entre 3 et 4%.

### Exemple 8.

Une solution de borane est obtenue par mélange de 0,48 g de borane B(C₆F₅)₃ hydraté à 18% dans l'isopropanol , 2,3 grammes de éthyl-3 (hydroxyméthyl-3)oxétane et 39 mg de produit Tinuvin 765®.

On ajoute 2,8 g de cette solution à 100 g d'oligomère de formule (I). Le mélange est stable à 20°C pendant plus de 4 heures; la réticulation est effectuée à 60°C pour donner un matériau de dureté Shore D égale à 50 après 24h.

### Exemple 9.

Dans un mélangeur de type Hauschild Speedmixer DAC150®, on introduit un pot en plastique contenant 12,5 g d'oligomère (I); 0,5 g de solution d'amorceur de l'exemple 8; 25 g de quartz; et 12,5 g de silice de combustion de surface spécifique 200m²/g.

Le mélangeur est mis en route successivement 3 fois 5 secondes après introduction du quartz et 3 fois 5 secondes au cours de l'introduction de la silice de combustion de telle façon que la température de mélange n'excède pas 40°C.

Le mélange est ensuite réticulé à 80°C pendant 10 minutes et on obtient un matériau de dureté Shore D supérieur à 90. Le retrait volumique est inférieur à 1%. (mesure par pycnométrie).

### Exemple 10.

Une solution de borane est obtenue par mélange de 0,48 g de borane B(C₆F₅)₃ hydraté à 18% dans l'isopropanol , 2,3 g de résine (**II**) et 39 mg de produit Tinuvin 765®.

On ajoute 2,8 g de cette solution à 100 g d'oligomère (**I**). Le mélange est stable à 20°C pendant plus de 4 heures; la réticulation est effectuée à 60°C pour donner un matériau de dureté Shore A supérieur à 90 et de dureté Shore D égale à 50 après 24h.

### Exemole11.

Un mélange bicomposant constitué d'une composition A et d'une composition B est préparé.

La composition A est préparée dans un mélangeur Hauschild Speedmixer DAC50 ® de 12,5 g d'oligomère (**I**) et 12,5 g de charge polyméthacrylate de méthyle en poudre (produit LUXASELF®). La composition B est constituée d'une solution d'amorceur selon l'exemple 10.

La composition A est ensuite mélangée à l'aide d'un petit mélangeur statique avec 2 g de la composition B. Le mélange obtenu est réticulé à température ambiante en 1 heure (ou en 10 minutes à 60°C).

## Revendications

1. Composition dentaire comprenant :
(**1**) 1 à 99 % en poids, et de préférence de 5 à 50%, d'au moins un oligomère ou polymère silicone réticulable et/ou polymérisable, liquide à température ambiante ou thermofusible à température inférieure à 100°C, et comprenant :
• au moins un motif de formule (FS) : 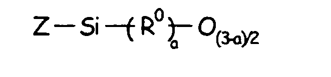 dans laquelle :
- a = 0, 1 ou 2,
- R⁰, identique ou différent, représente un radical alkyle, cycloalkyle, aryle, vinyle, hydrogéno, alcoxy, de préférence un alkyle inférieur en C₁-C₆,
- Z, identique ou différent, est un substituant organique comportant au moins une fonction réactive époxy, et/ou alcénylether et/ou oxétane et/ou dioxolane et/ou carbonate, et de préférence Z étant un substituant organique comportant au moins une fonction réactive époxy, et/ou dioxolane,
• et au moins deux atomes de silicium;
(**2**) de 5 à 90 % en poids, et de préférence 10 à 80 %, d'au moins une charge dentaire;
(**3**) de 0,001 à 5% en poids d'au moins un amorceur de polymérisation et/ou de réticulation activable thermiquement comprenant un dérivé du bore de formule (I) et/ou sa ou ses formes solvatées:
(A)ₓB(R')_{y} (I)
dans laquelle:
· les symboles R' sont identiques ou différents et représentent :
- un radical alkoxy en C₁-C₁₂, de préférence en C₁-C₈, linéaire ou ramifié, éventuellement substitué par au moins un élément électroattracteur, notamment un atome d'halogène (le fluor tout particulièrement), ou un groupement électroattracteur comme par exemple les groupements CF₃, NO₂, CN,
- un radical phényle substitué par au moins (i) un élément électroattracteur, notamment un atome d'halogène (fluor tout particulièrement), (ii) un groupement électroattracteur, notamment un groupement CF₃, NO₂, CN, ou (iii) un radical hydrocarboné en C₁-C₁₂, de préférence en C₁-C₈, linéaire, ramifié, mono- ou poly- cyclique, saturé, insaturé ou aromatique et de préférence substitué par au moins un élément atome d'halogène ou un radical alkyle en C₁-C₁₂, linéaire ou ramifié, mono- poly- ou per-halogéné, et
- un radical aryle contenant au moins deux noyaux aromatiques tel que biphényle, naphtyle, éventuellement substitué par au moins un élément électroattracteur, notamment atome d'halogène (fluor tout particulièrement), ou un groupement électroattracteur notamment un groupement CF₃, NO₂, CN,
- deux groupements R' peuvent être liés entre eux de manière à constituer avec l'atome de bore auquel ils sont liés un cycle à 5 ou 10 atomes avec ledit cycle pouvant être saturé, insaturé ponté et/ou aromatique et comprendre un ou plusieurs hétéroatomes choisis parmi les atomes d'hydrogène, d'azote et de bore avec l'atome bore présent dans ledit cycle pouvant lui-même être substitué par un radical tel que défini pour A ou R' en formule générale (I) ;
· les symboles A représentent indépendamment l'un de l'autre :
- un atome d'halogène et/ou
- un radical hydroxyle;
· x représente 0 ou l'entier 1 ou 2 et y l'entier 1, 2 ou 3 avec la somme de x+y étant égale à 3.

2. Composition selon la revendication 1, **caractérisée en ce que** les symboles R' sont choisis de manière à conférer à l'atome de bore auquel ils sont liés un encombrement stérique suffisant pour lui assurer une protection efficace contre des phénomènes d'oxydation et/ou d'hydratation.

3. Composition selon l'une quelconque des revendications précédentes, **caractérisé en ce que** les symboles R' contribuent globalement avec les symboles A à un σₚ au moins égal à celui de 3 radicaux (C₆H₄F).

4. Composition selon l'une quelconque des revendications précédentes, **caractérisée en ce qu'**il répond à la formule générale (la) 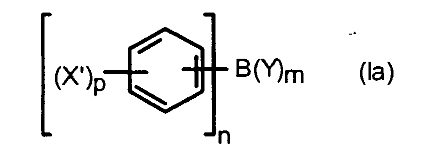 dans laquelle:
- n représente un nombre entier compris entre 1 et 3 et m un nombre entier compris entre 0 et 2 avec la somme de n et m étant égale à 3,
- les symboles Y sont identiques ou différents et représentent
a) un groupement hydroxyle,
b) un atome d'halogène,
c) un radical alkoxy en C₁-C₁₂, de préférence en C₁-C₈, linéaire ou ramifié, de préférence substitué par au moins un élément électroattracteur comme un atome d'halogène et en particulier un atome de fluor,
d) deux groupements Y peuvent être liés entre eux de manière à constituer avec l'atome de bore auquel ils sont liés un cycle en C₅-C₁₀ avec ledit cycle pouvant être saturé, insaturé, ponté et/ou aromatique et comprendre un ou plusieurs hétéroatomes choisis parmi les atomes d'oxygène, d'azote et de bore avec l'atome bore présent dans ledit cycle pouvant lui-même être substitué par un radical tel que défini pour Y en formule générale (la) et
- les symboles X' sont identiques ou différents et représentent
- un atome d'halogène,
- un radical hydrocarboné en C₁-C₁₂, de préférence en C₁-C₈, linéaire, ramifié, mono- ou poly- cyclique, saturé, insaturé ou aromatique et de préférence substitué par au moins un élément atome d'halogène ou un radical alkyle en C₁-C₁₂, linéaire ou ramifié, mono- poly- ou per-halogéné, et
- les indices p sont identiques ou différents et représentent un entier compris entre 0 et 5, avec de préférence au moins l'un des symboles p supérieur à 3 et plus préférentiellement égal à 5.

5. Composition selon l'une quelconque des revendications précédentes **caractérisée en ce que** Z est un substituant organique Z1 comportant au moins une fonction réactive époxy, et/ou dioxolane, et de préférence au moins une fonction réactive époxy.

6. Composition selon l'une quelconque des revendications précédentes **caractérisée en ce que** l'oligomère ou polymère (1) comporte en outre d'autres fonctions réactives Z telles que les fonctions réactives Z2 alcénylether, oxétane et/ou carbonate.

7. Composition selon l'une quelconque des revendications précédentes, **caractérisée en ce que** la ou les fonctions réactives de Z1 sont choisies parmi les radicaux suivants :

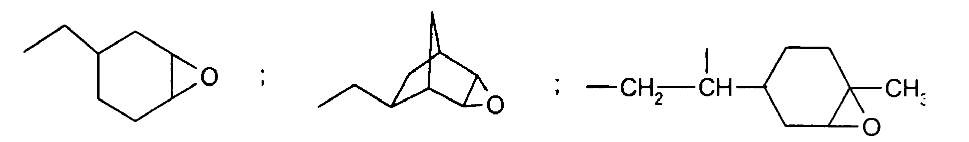

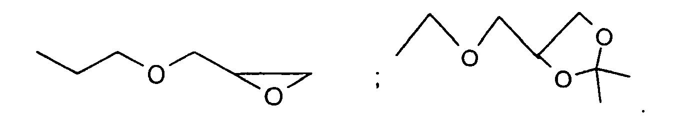

8. Composition dentaire selon l'une quelconque des revendications précédentes **caractérisée en ce que** la composition dentaire comprend en outre des monomères, oligomères et/ou polymères réticulables et/ou polymérisables de nature organique.

9. Composition dentaire selon l'une quelconque des revendications précédentes **caractérisée en ce qu'**elle comprend au moins un composé stabilisant à fonctions amines, et de préférence une amine encombrée stériquement.

10. Utilisation d'une composition dentaire selon l'une quelconque des revendications précédentes pour la réalisation de prothèses dentaires.

11. Utilisation d'une composition dentaire selon l'une quelconque des revendications précédentes 1 à 9 pour la réalisation de matériau de restauration dentaire.

12. Utilisation d'une composition dentaire selon l'une quelconque des revendications précédentes 1 à 9 pour la réalisation de dent artificielle.

13. Prothèse dentaire susceptible d'être obtenue à partir d'une composition selon l'une quelconque des revendications 1 à 9.

14. Matériau de restauration dentaire susceptible d'être obtenu à partir d'une composition selon l'une quelconque des revendications 1 à 9.

15. Dent artificielle susceptible d'être obtenue à partir d'une composition selon l'une quelconque des revendications 1 à 9.

## Claims

1. Dental composition comprising:
**(1)** 1 to 99% by weight, and preferably from 5 to 50%, of at least one crosslinkable and/or polymerizable silicone oligomer or polymer which is liquid at room temperature or which is heat-meltable at a temperature of less than 100°C, and which comprises:
• at least one unit of formula (FS) : 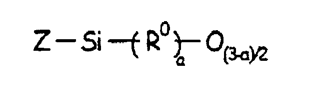 in which:
- a = 0, 1 or 2,
- R⁰, identical or different, represents an alkyl, cycloalkyl, aryl, vinyl, hydrogeno or alkoxy radical, preferably a C₁-C₆ lower alkyl,
- Z, identical or different, is an organic substituent comprising at least one epoxy and/or alkenyl ether and/or oxetane and/or dioxolane and/or carbonate reactive functional group, and preferably Z being an organic substituent comprising at least one epoxy and/or dioxolane reactive functional group,
• and at least two silicon atoms;
**(2)** from 5 to 90% by weight, and preferably 10 to 80%, of at least one dental filler;
**(3)** from 0.001 to 5% by weight of at least one heat-activatable polymerization and/or crosslinking initiator comprising a boron derivative of formula (I) and/or its solvated form(s):
(A)ₓB(R')_{y} (I)
in which:
• the symbols R' are identical or different and represent:
- a linear or branched C₁₋₁₂, preferably C₁-C₈, alkoxy radical, optionally substituted with at least one electron-attracting element, in particular a halogen atom (fluorine most particularly), or an electron-attracting group such as for example the CF₃, NO₂ and CN groups,
- a phenyl radical substituted with at least (i) an electron-attracting element, in particular a halogen atom (fluorine most particularly), (ii) an electron-attracting group, in particular a CF₃, NO₂ or CN group, or (iii) a saturated, unsaturated or aromatic, mono- or polycyclic, linear or branched C₁-C₁₂, preferably C₁-C₈, hydrocarbon radical, preferably substituted with at least one element which is a halogen atom or a mono-, poly- or perhalogenated, linear or branched C₁-C₁₂ alkyl radical, and
- an aryl radical containing at least two aromatic rings such as biphenyl, naphthyl, optionally substituted with at least one electron-attracting element, in particular a halogen atom (fluorine most particularly), or an electron-attracting group, in particular a CF₃, NO₂ or CN group,
- two groups R' may be linked to each other so as to constitute, with the boron atom to which they are attached, a 5- or 10-atom ring, it being possible for said ring to be saturated, unsaturated bridged and/or aromatic and to comprise one or more heteroatoms chosen from hydrogen, nitrogen and boron atoms, it being possible for the boron atom present in said ring to be substituted itself with a radical as defined for A or R' in general formula (I);
• the symbols A represent independently of each other:
- a halogen atom, and/or
- a hydroxyl radical;
• x represents 0 or the integer 1 or 2 and y the integer 1, 2 or 3 with the sum of x+y being equal to 3.

2. Composition according to Claim 1, **characterized in that** the symbols R' are chosen so as to confer on the boron atom to which they are attached a steric hindrance sufficient to ensure effective protection against oxidation and/or hydration phenomena.

3. Composition according to either of the preceding claims, **characterized in that** the symbols R' contribute overall with the symbols A to a σₚ at least equal to that of 3 radicals (C₆H₄F).

4. Composition according to any one of the preceding claims, **characterized in that** it corresponds to general formula (Ia): 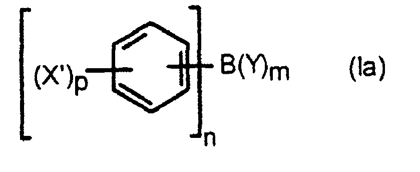 in which:
- n represents an integer between 1 and 3 and m an integer between 0 and 2 with the sum of n and m being equal to 3,
- the symbols Y are identical or different and represent
a) a hydroxyl group,
b) a halogen atom,
c) a linear or branched C₁-C₁₂, preferably C₁-C₈, alkoxy radical, preferably substituted with at least one electron-attracting element such as a halogen atom and in particular a fluorine atom,
d) two groups Y may be linked to each other so as to constitute with the boron atom to which they are attached a C₅-C₁₀ ring with said ring being possibly saturated, unsaturated, bridged and/or aromatic and possibly comprising one or more heteroatoms chosen from oxygen, nitrogen and boron atoms, it being possible for the boron atom present in said ring to be substituted itself with a radical as defined for Y in general formula (Ia) and
- the symbols X' are identical or different and represent
- a halogen atom,
- a saturated, unsaturated or aromatic, mono- or polycyclic, linear or branched C₁-C₁₂, preferably C₁-C₈, hydrocarbon radical, preferably substituted with at least one element which is a halogen atom or a mono-, poly- or perhalogenated, linear or branched C₁-C₁₂ alkyl radical, and
- the indices p are identical or different and represent an integer between 0 and 5, with preferably at least one of the symbols p being greater than 3 and more preferably equal to 5.

5. Composition according to any one of the preceding claims, **characterized in that** Z is an organic substituent Z1 comprising at least one epoxy and/or dioxolane reactive functional group, and preferably at least one epoxy reactive functional group.

6. Composition according to any one of the preceding claims, **characterized in that** the oligomer or polymer (1) additionally comprises other reactive functional groups Z such as the alkenyl ether, oxetane and/or carbonate reactive functional groups Z2.

7. Composition according to any one of the preceding claims, **characterized in that** the reactive functional group(s) of Z1 are chosen from the following radicals:

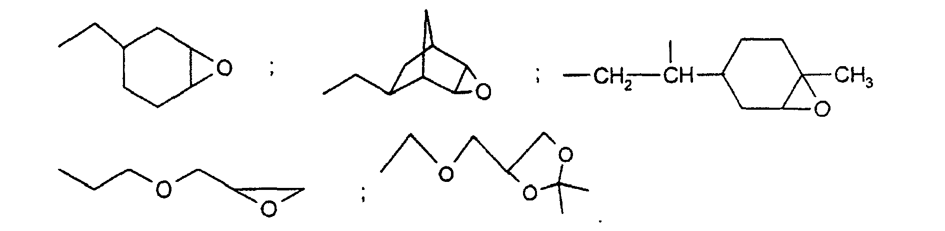

8. Dental composition according to any one of the preceding claims, **characterized in that** the dental composition additionally comprises crosslinkable and/or polymerizable monomers, oligomers and/or polymers of an organic nature.

9. Dental composition according to any one of the preceding claims, **characterized in that** it comprises at least one stabilizing compound with amine functional groups, and preferably a sterically hindered amine.

10. Use of a dental composition according to any one of the preceding claims, for producing dental prostheses.

11. Use of a dental composition according to any one of the preceding Claims 1 to 9, for producing dental restoration material.

12. Use of a dental composition according to any one of the preceding Claims 1 to 9, for producing an artificial tooth.

13. Dental prosthesis, which can be obtained from a composition according to any one of Claims 1 to 9.

14. Dental restoration material, which can be obtained from a composition according to any one of Claims 1 to 9.

15. Artificial tooth, which can be obtained from a composition according to any one of Claims 1 to 9.

## Patentansprüche

1. Dentalzusammensetzung, umfassend:
(1) 1 bis 99 Gew.-%, und vorzugsweise 5 bis 50 Gew.-%, von mindestens einem vernetzbaren und/oder polymerisierbaren Silicon-Oligomer oder Silicon-Polymer, das bei Umgebungstemperatur flüssig oder bei einer Temperatur von unter 100 °C thermoschmelzbar ist, und umfassend:
• mindestens eine Struktureinheit der Formel (FS) 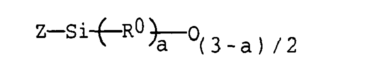 in der
- a gleich 0, 1 oder 2 ist,
- R⁰, gleich oder verschieden, einen Rest Alkyl, Cycloalkyl, Aryl, Vinyl, Hydrogeno, Alkoxy, und vorzugsweise niederes Alkyl mit 1 bis 6 Kohlenstoffatomen darstellt,
- Z, gleich oder verschieden, ein organischer Substituent ist, umfassend mindestens eine reaktive Funktion Epoxy und/oder Alkenylether und/oder Oxethan und/oder Dioxolan und/oder Carbonat, und vorzugsweise ist Z ein organischer Substituent, der mindestens eine reaktive Funktion Epoxy und/oder Dioxolan umfasst,
• und mindestens zwei Siliciumatome;
(2) 5 bis 90 Gew.-%, und vorzugsweise 10 bis 80 Gew.-%, von mindestens einem Dentalfüllstoff;
(3) 0,001 bis 5 Gew.-% von mindestens einem thermisch aktivierbaren Initiator für die Polymerisation und/oder die Vernetzung, umfassend ein Borderivat der Formel (I) und/oder seine solvatisierte(n) Form(en)
(A)ₓB(R')_{y} (I)
in der
. die Symbole R' gleich oder verschieden sind und darstellen:
- einen linearen oder verzweigten Rest Alkoxy mit 1 bis 12 Kohlenstoffatomen, vorzugsweise 1 bis 8 Kohlenstoffatomen, gegebenenfalls substituiert durch mindestens ein Elektronen anziehendes Element, insbesondere ein Halogenatom (ganz besonders Fluor), oder eine Elektronen anziehende Gruppe, wie beispielsweise die Gruppen CF₃, NO₂, CN,
- einen Rest Phenyl, substituiert durch mindestens (i) ein Elektronen anziehendes Element, insbesondere ein Halogenatom (ganz besonders Fluor), (ii) eine Elektronen anziehende Gruppe, insbesondere eine Gruppe CF₃, NO₂, CN, oder (iii) einen linearen oder verzweigten, mono- oder polycyclischen, gesättigten, ungesättigten oder aromatischen Kohlenwasserstoff-Rest mit 1 bis 12 Kohlenstoffatomen, vorzugsweise 1 bis 8 Kohlenstoffatomen, und vorzugsweise substituiert durch mindestens ein Element Halogenatom oder einen linearen oder verzweigten, mono,- poly-oder perhalogenierten Rest Alkyl mit 1 bis 12 Kohlenstoffatomen, und
- einen Rest Aryl, enthaltend mindestens zwei aromatische Kerne wie Biphenyl, Naphthyl, gegebenenfalls substituiert durch mindestens ein Elektronen anziehendes Element insbesondere ein Halogenatom (ganz besonders Fluor), oder eine Elektronen anziehende Gruppe, insbesondere eine Gruppe CF₃, NO₂, CN,
- wobei zwei Gruppen R' untereinander verbunden sein können, so dass sie mit dem Boratom, an das sie gebunden sind, einen Ring mit 5 oder 10 Atomen bilden, und der genannte Ring als Brücke gesättigt, ungesättigt und/oder aromatisch sein und ein oder mehrere Heteroatome umfassen kann, ausgewählt unter den Atomen von Wasserstoff, Stickstoff und Bor, wobei das in dem genannten Ring anwesende Boratom selbst substituiert sein kann durch einen wie bei A oder R' in der allgemeinen Formel (I) definierten Rest;
. die Symbole A unabhängig voneinander darstellen:
- ein Halogenatom und/oder
- einen Rest Hydroxyl;
. x stellt 0 oder das Ganze 1 oder 2 und y das Ganze 1, 2 oder 3 dar, mit der Summe von x+y gleich 3.

2. Zusammensetzung nach Anspruch 1, **dadurch gekennzeichnet, dass** die Symbole R' in der Weise ausgewählt werden, dass sie dem Boratom, an das sie gebunden sind, eine sterische Hinderung verleihen, die ausreichend ist, um ihm einen wirksamen Schutz gegenüber Erscheinungen von Oxidation und/oder Hydratisierung zu gewährleisten.

3. Zusammensetzung nach irgendeinem der vorstehenden Ansprüche, **dadurch gekennzeichnet, dass** die Symbole R' global mit den Symbolen A zu einem σₚ von mindestens gleich dem der drei Reste (C₆H₄F) beitragen.

4. Zusammensetzung nach irgendeinem der vorstehenden Ansprüche, **dadurch gekennzeichnet, dass** sie der allgemeinen Formel (Ia) 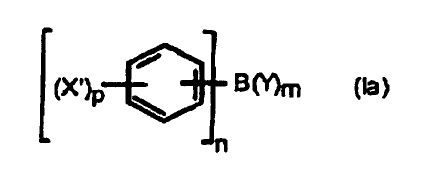 entspricht, in der
- n eine ganze Zahl zwischen einschließlich 1 und 3 darstellt und m eine ganze Zahl zwischen einschließlich 0 und 2 darstellt, mit der Summe von n und m gleich 3,
- die Symbole Y gleich oder verschieden sind und darstellen:
a) eine Gruppe Hydroxyl,
b) ein Halogenatom,
c) einen linearen oder verzweigten Rest Alkoxy mit 1 bis 12 Kohlenstoffatomen, vorzugsweise 1 bis 8 Kohlenstoffatomen, vorzugsweise substituiert durch mindestens ein Elektronen anziehendes Element wie ein Halogenatom und insbesondere ein Fluoratom,
d) wobei zwei Gruppen Y untereinander verbunden sein können, so dass sie mit dem Boratom, an das sie gebunden sind, einen Ring mit 5 bis 10 Kohlenstoffatomen bilden, und der genannte Ring als Brücke gesättigt, ungesättigt und/oder aromatisch sein und ein oder mehrere Heteroatome umfassen kann, ausgewählt unter den Atomen von Sauerstoff, Stickstoff und Bor, wobei das in dem genannten Ring anwesende Boratom selbst substituiert sein kann durch einen wie bei Y in der allgemeinen Formel (Ia) definierten Rest;
- die Symbole X' gleich oder verschieden sind und darstellen:
. ein Halogenatom,
. einen linearen oder verzweigten, mono- oder polycyclischen, gesättigten, ungesättigten oder aromatischen Kohlenwasserstoff-Rest mit 1 bis 12 Kohlenstoffatomen, vorzugsweise 1 bis 8 Kohlenstoffatomen, und vorzugsweise substituiert durch mindestens ein Element Halogenatom oder einen linearen oder verzweigten, mono,- poly- oder perhalogenierten Rest Alkyl mit 1 bis 12 Kohlenstoffatomen, und
- die Indices p gleich oder verschieden sind und ein Ganzes zwischen einschließlich 0 und 5 darstellen, wobei vorzugsweise mindestens eines der Symbole p über 3 und noch mehr bevorzugt gleich 5 beträgt.

5. Zusammensetzung nach irgendeinem der vorstehenden Ansprüche, **dadurch gekennzeichnet, dass** Z ein organischer Substituent Z1 ist, umfassend mindestens eine reaktive Funktion Epoxy und/oder Dioxolan, und vorzugsweise mindestens eine reaktive Funktion Epoxy.

6. Zusammensetzung nach irgendeinem der vorstehenden Ansprüche, **dadurch gekennzeichnet, dass** das Oligomer oder Polymer (1) außerdem andere reaktive Funktionen Z umfasst, wie die reaktiven Funktionen Z2 Alkenylether, Oxethan und/oder Carbonat.

7. Zusammensetzung nach irgendeinem der vorstehenden Ansprüche, **dadurch gekennzeichnet, dass** die reaktive(n) Funktion(en) von Z1 unter den folgenden Resten ausgewählt werden:

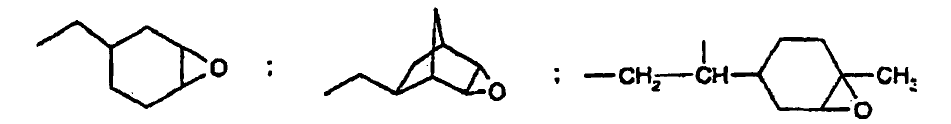

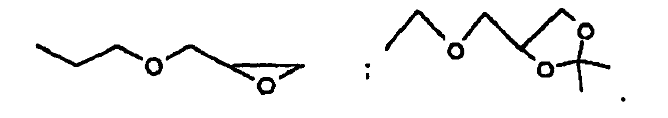

8. Dentalzusammensetzung nach irgendeinem der vorstehenden Ansprüche, **dadurch gekennzeichnet, dass** die Dentalzusammensetzung außerdem vernetzbare und/oder polymerisierbare Monomere, Oligomere und/oder Polymere von organischer Beschaffenheit umfasst.

9. Dentalzusammensetzung nach irgendeinem der vorstehenden Ansprüche, **dadurch gekennzeichnet, dass** sie mindestens eine stabilisierende Verbindung mit Aminfunktionen umfasst und vorzugsweise ein sterisch gehindertes Amin.

10. Verwendung einer Dentalzusammensetzung nach irgendeinem der vorstehenden Ansprüche für die Herstellung von Zahnprothesen.

11. Verwendung einer Dentalzusammensetzung nach irgendeinem der vorstehenden Ansprüche 1 bis 9 für die Herstellung von Material zur Zahnrestaurierung.

12. Verwendung einer Dentalzusammensetzung nach irgendeinem der vorstehenden Ansprüche 1 bis 9 für die Herstellung von künstlichen Zähnen.

13. Zahnprothese, die geeignet ist, ausgehend von einer Zusammensetzung nach irgendeinem der Ansprüche 1 bis 9 erhalten zu werden.

14. Material zur Zahnrestaurierung, das geeignet ist, ausgehend von einer Zusammensetzung nach irgendeinem der Ansprüche 1 bis 9 erhalten zu werden.

15. Künstlicher Zahn, der geeignet ist, ausgehend von einer Zusammensetzung nach irgendeinem der Ansprüche 1 bis 9 erhalten zu werden.
